# FASCICULE DE BREVET EUROPEEN

(11) **EP 0 605 284 B1**
(45) Date de publication et mention de la délivrance du brevet: **08.01.1997**
(21) Numéro de dépôt: 93403096.6
(22) Date de dépôt: 20.12.1993
(51) Int. Cl.: A61K 7/02, A61K 7/00, A61K 7/035

(54) **Composition solide ou pateuse de maquillage et procédé de préparation**
Feste oder pastöse Schminke und Herstellungsverfahren
Solid or pasty makeup and preaparation process

(30) Priorité: 24.12.1992 FR 9215728
(43) Date de publication de la demande: 06.07.1994
(73) Titulaire: L'OREAL, 75008 Paris (FR)
(72) Inventeur: Pradier, Francois, F-92260 Fontenay-aux-Roses (FR); Maison-Belhomme, Elisabeth, F-94300 Vincennes (FR); Felardos, Christian, F-94550 Chevilly Larue (FR)
(74) Mandataire: Peuscet, Jacques

(56) Documents cités:
- EP-A- 0 157 680
- EP-A- 0 195 702
- EP-A- 0 254 612
- EP-A- 0 447 286
- EP-A- 0 486 394
- FR-A- 1 586 848
- GB-A- 2 027 341
- PATENT ABSTRACTS OF JAPAN vol. 15, no. 189 (C-831)15 Mai 1991 & JP-A-30 047 110 (KOBAYASHI KOSE CO.)

## Description

La présente invention concerne une composition de maquillage contenant une proportion élevée de poudre légère et un procédé pour la fabrication de ces compositions.

Il est connu de préparer des compositions solides ou pâteuses de maquillage de deux types contenant des produits pulvérulents :
- les compositions coulées qui sont préparées en mélangeant une phase grasse à l'état fondu avec des produits pulvérulents, avec ou sans solvant volatil ;
- les compositions compactées, qui sont préparées en mélangeant un produit pulvérulent avec un liant gras et en soumettant le mélange à une compression.

De façon classique, on prépare ces deux types de compositions avec des poudres ayant une densité supérieure à environ 0,5. Il serait désirable d'introduire dans ces compositions de maquillage des poudres légères, c'est-à-dire de densité au plus égale à 0,07 pour améliorer leur toucher. Mais, jusqu'à présent, il n'était possible d'introduire que des quantités limitées de poudres légères, ces quantités ne dépassant pas généralement environ 4,5 % en poids de la composition. En effet, au delà de cette proportion limitée, dans le cas des compositions coulées sans solvant volatil, il devient difficile voire impossible, avec les procédés usuels d'homogénéiser le mélange de phase grasse et de produits pulvérulents et, par conséquent, le mélange obtenu est difficile, voire impossible, à couler. Dans le cas des compositions compactées, l'introduction de quantités importantes de produits pulvérulents légers entraîne aussi une difficulté d'homogénéisation. Il était donc impossible jusqu'à ce jour d'obtenir des compositions homogènes utilisables pour le maquillage avec une forte proportion de poudres légères. EP-A-447 286 décrit une poudre compactée comprenant un liant gras, des microsphères creuses constituant une poudre légère et d'autres charges sous la forme de poudre solide. Mais, dans ces compositions, la quantité de phase grasse est inférieure à 25 % en poids, alors qu'il serait souhaitable pour le maquillage d'avoir une phase grasse beaucoup plus importante.

Dans EP-A-254 612, on décrit une poudre non compactée utilisable pour le maquillage ; cette poudre comprend une huile, des microsphères creuses constituant une poudre légère ainsi que d'autres constituants de la phase pulvérulente. Mais ce document ne décrit pas une composition solide ou pâteuse de maquillage mais une poudre libre d'utilisation tout-à-fait différente.

EP-A-486 394 décrit une poudre coulée comprenant un liant gras, des microsphères creuses correspondant à une poudre légère, ainsi que d'autres charges pulvérulentes. Mais ce document ne permet pas d'obtenir une composition comportant à la fois une phase grasse importante et une proportion élevée de poudre légère dans la phase pulvérulente.

Selon la présente invention, on a trouvé qu'en utilisant pour la préparation des compositions coulées sans solvant ou compactées, un mélangeur extrudeur-cuiseur à une ou deux vis, il était possible d'obtenir des compositions globalement homogènes utilisables pour le maquillage contenant jusqu'à 30 % en poids de poudre de densité au plus égale à 0,07.

La présente invention a donc pour objet une composition solide ou pâteuse de maquillage, obtenue sous forme homogène par extrusion contenant une phase grasse et une phase pulvérulente, la phase pulvérulente étant au moins partiellement constituée d'une poudre légère, caractérisée par le fait que la poudre légère a une densité au plus égale à O,O7 et représente de 5 à 30 % en poids par rapport au poids total de la composition, le rapport pondéral de la phase pulvérulente totale à la poudre légère étant compris entre 5 et 16 et que la phase grasse représente de 20 à 70 % en poids par rapport au poids total de la composition.

Selon l'invention, la phase grasse représente, de préférence, de 20 à 50 % en poids de la composition, la phase pulvérulente représentant, par conséquent, de 50 à 80 % en poids de la composition, ce qui correspond à la fabrication de compositions de maquillage du type coulé ou du type compacté.

La poudre légère de densité au plus égale à 0,07 peut comporter au moins un des constituants suivants :
- les microsphères creuses en matériau thermoplastique préparées par des procédés connus, tels ceux décrits dans US-A-3 615 972 et EP-A-0 56219. Ces microsphères peuvent être réalisées en tous matériaux thermoplastiques non toxiques et non irritants, par exemple en polymères ou copolymères de dérivés éthyléniques tels que polyéthylène, polystyrène, copolymère chlorure de vinyle-acrylonitrile ou polyacrylonitrile, en polyamides, en polyesters, en polymères urée-formaldéhyde ou en copolymères de chlorure de vinylidène (tels que le chlorure de vinylidène-acrylonitrile). On peut citer notamment les microsphères commercialisées sous la dénomination commerciale "EXPANCEL®" par la société "KEMANORD PLAST" ou sous la dénomination commerciale "MICROPEARL F 80 ED" par la société "MATSUMOTO".
- la poudre de silice, par exemple celle commercialisée sous la dénomination commerciale "CAB-0-SIL® TS-530" par la société "CABOT".

La phase pulvérulente contient avantageusement des poudres ayant une densité plus élevée que celle de la poudre légère. Ces poudres peuvent être toute poudre connue utilisée de façon usuelle pour la préparation de compositions de maquillage, ces poudres pouvant comporter au moins un pigment et/ou au moins une charge.

Les pigments peuvent être choisis parmi les pigments minéraux, les pigments organiques et les pigment nacrés.

Parmi les pigments minéraux, on peut citer, à titre d'exemples :
- le dioxyde de titane (rutile ou anatase), éventuellement traité en surface et codifié dans le "Color Index" (CI) sous la référence CI 77891 ;
- les oxydes de fer noir, jaune, rouge et brun (CI 77499, 77492, 77491) ;
- le violet de manganèse (CI 77742) ;
- le bleu outremer (CI 77007) ;
- l'oxyde de chrome (CI 77288) ;
- l'oxyde de chrome hydraté (CI 77289) et
- le bleu ferrique (CI 77510).

Parmi les pigments organiques, on peut citer, par exemple, les pigments : D & C red n° 19 (CI 45170) ; D & C red n° 9 (CI 15585) ; D & C red n° 21 (CI 45380) ; D & C orange n° 4 (CI 15510) ; D & C orange n° 5 (CI 45370) ; D & C red n° 27 (CI 45410) ; D & C red n° 13 (CI 15630) ; D & C red n° 7 (CI 15850:1) ; D & C red n° 6 (CI 15850:2) ; D & C yellow n° 5 (CI 19140) ; D & C red n° 36 (CI 12085) ; D & C orange n° 10 (CI 45425) ; D & C yellow n° 6 (CI 15985) ; D & C red n° 30 (CI 73360) ; D & C red n° 3 (CI 45430) ; le noir de carbone (CI 77266) et les laques à base de carmin de cochenille (CI 75470) ;

Les pigments nacrés peuvent être choisis notamment parmi :
- les pigments nacrés blancs, tels que le mica recouvert d'oxyde de titane, l'oxychlorure de bismuth ; et
- les pigments nacrés colorés, tel que le mica titane avec des oxydes de fer, le mica titane avec du bleu ferrique ou de l'oxyde de chrome, le mica titane avec un pigment organique du type précité, ainsi que les pigments à base d'oxychlorure de bismuth.

Ces pigments peuvent représenter jusqu'à 30 % en poids par rapport au poids total de la composition.

Les charges sont choisies notamment parmi :
- le talc, qui est un silicate de magnésium hydraté, utilisé sous forme de particules généralement de dimensions inférieures à 40 µm ; le talc possède des propriétés absorbantes de l'humidité et est utilisé surtout en raison de son toucher onctueux ;
- les micas, qui sont des aluminosilicates de compositions variées, qui se présentent sous forme d'écailles ayant des dimensions de 2 à 200 µm, de préférence de 5 à 70 µm et une épaisseur de 0,1 à 5 µm, de préférence de 0,2 à 3 µm ; les micas peuvent être d'origine naturelle (par exemple muscovite, margarite, roscoelithe, lipidolithe, biotite) ou d'origine synthétique ; les micas sont généralement transparents et permettent de conférer à la peau un aspect satiné ;
- l'amidon, en particulier l'amidon de riz ;
- le kaolin, qui est un silicate d'aluminium hydraté, qui se présente sous la forme de particules de formes isotropes ayant des dimensions généralement inférieures à 30 µm, et qui possède de bonnes propriétés d'absorption des corps gras ;
- les oxydes de zinc et de titane, généralement utilisés sous la forme de particules ayant des dimensions ne dépassant pas quelques micromètres (ou même inférieures à 1 µm dans le cas de l'oxyde de titane) ; ces oxydes ont un toucher onctueux, ont un bon pouvoir couvrant et ont une opacité importante ;
- le carbonate de calcium précipité qui, sous forme de particules de dimensions inférieures à 10 µm, a un toucher onctueux et permet d'obtenir un aspect mat ;
- le carbonate et l'hydrocarbonate de magnésium, qui possèdent notamment des propriétés de fixation des parfums ;
- la silice ;
- le dioxyde de titane ;
- des savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium ; ces savons, présents généralement sous la forme de particules ayant des dimensions inférieures à 10 µm, ont un toucher onctueux et facilitent l'adhérence de la poudre sur la peau ;
- les poudres de polymères synthétiques non expansées, tels que le polyéthylène, les polyesters (par exemple isophtalate ou téréphtalate de polyéthylène), et les polyamides (par exemple le nylon), sous la forme de particules ayant des dimensions inférieures à 50 µm, qui possèdent des propriétés absorbantes et permettent de conférer à la peau un aspect velouté.

On utilise plus particulièrement les poudres minérales telles que la silice sphérique, les dioxydes de titane sphériques comme le "SPHERITITAN®" (marque déposée), les billes de verre et de céramique commercialisées par la société "3M" sous les dénominations commerciales "MACROLITES" ; des poudres de matériaux organiques d'origine naturelle comme les amidons de maïs, de blé, de riz, réticulés ou non ; des poudres de polymères de synthèse (éventuellement réticulés) sphéronisées, comme des poudres de polyamides (par exemple de nylon ou de poly-β-alanine), de polyéthylène, d'acides poly-méthacryliques, de polystyrène (réticulé par le divinylbenzène), de résine de silicone, de téflon (par exemple : "FLUON®", particules commercialisées par la société "MONTEFLUOS" ; "HOSTAFLONQ", particules commercialisées par la société "HOECHST").

Ces charges peuvent représenter jusqu'à 80 % en poids par rapport au poids total de la composition.

Pigments et charges peuvent être enrobés par des substances telles que des acides aminés, des silicones, des sels métalliques ou du collagène, notamment afin de modifier leur état de surface.

La phase grasse est constituée d'au moins un liant gras, liquide ou solide à température ambiante, et/ou d'au moins un polymère synthétique oléosoluble dont l'utilisation dans les cosmétiques est connue.

Parmi les liants, on peut citer, notamment, les huiles ou corps gras d'origine animale, végétale, minérale ou synthétique, les cires ou leurs mélanges.

Les huiles ou corps gras sont, en particulier, choisis parmi l'huile de vison, l'huile de tortue, l'huile de soja, l'huile de pépins de raison, l'huile de sésame, l'huile de maïs, l'huile de colza, l'huile de tournesol, l'huile de coton, l'huile d'avocat, l'huile d'olive, l'huile de ricin, l'huile de jojoba ou l'huile d'arachide ; une huile d'hydrocarbures, telles que les huiles de paraffine, le squalane, la vaseline ; des esters tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyldécyle, le palmitate de 2-octyl-décyle, le myristate de 2-octyl-dodécyle, le succinate de 2-di-éthyl-héxyle, le malate de diisostéaryle, le lactate de 2-octyl-dodécyle, le triisostéarate de glycérine ou le triisostéarate de diglycérine ; une huile de silicone comme les polyméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées ; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique ou l'acide isostéarique ; des alcools gras supérieurs tels que le cétanol, l'alcool stéarylique ou l'alcool oléique.

Les cires peuvent être choisies dans le groupe formé par les cires animales, les cires végétales, les cires minérales, les cires synthétiques et les fractions diverses de cires naturelles. Parmi les cires animales utilisables, on peut citer les cires d'abeille, les cires de lanoline et les cires d'insectes de Chine. Parmi les cires végétales, on peut citer la cire de carnauba, de candélila, d'ouricurry, les cires de fibres de liège, les cires de canne à sucre, les cires du Japon, les cires de jojoba hydrogénées et les huiles hydrogénées qui sont obtenues par hydrogénation catalytique de corps gras composés de chaîne grasse, linéaire ou non, en C₈-C₃₂ et qui ont les qualités correspondant à la définition des cires. On peut citer, en particulier, l'huile de tournesol hydrogénée, l'huile de ricin hydrogénée, l'huile de coprah hydrogénée et la lanoline hydrogénée. Parmi les cires minérales, on peut citer les paraffines, les cires microcristallines, les cires de Montan et les ozokérites. Parmi les cires synthétiques, on peut citer les cires de polyéthylène, les cires obtenues par la synthèse de Fisher et Tropsch, les copolymères cireux ainsi que leurs esters, et les cires de silicone telles que les polyalcoxy et polyalkylsiloxanes.

La phase grasse peut, de façon connue, contenir au moins un actif cosmétique lipophile.

D'autres ingrédient divers peuvent être introduits dans les compositions selon l'invention, tels que des agents astringents, qui sont utilisés dans les poudres désodorisantes ou dans les poudres pour les pieds, tels que l'hydroxychlorure d'aluminium ou les aluns ; des filtres solaires ; des agents adoucissants ; des agents hydratants (par exemple, sorbitol, glycérine) ; des agents cicatrisants ; des agents anti-radicaux libres ; des vitamines ; des parfums.

Ces ingrédients peuvent représenter jusqu'à 5 % en poids par rapport au poids total de la composition.

La présente invention a également pour objet un procédé de préparation de la composition décrite ci-dessus.

La présente invention a donc pour objet un procédé de préparation d'une composition cosmétique homogène constituée d'une phase grasse et d'une phase pulvérulente contenant de 5 à 30 % en poids de poudre légère ayant une densité au plus égale à 0,07, caractérisé par le fait qu'on effectue le mélange dans un mélangeur extrudeur-cuiseur comportant une ou deux vis.

Les mélangeurs extrudeur-cuiseur utilisables selon l'invention sont des appareils de type connu, mis en oeuvre notamment de façon courante dans l'industrie alimentaire et l'industrie chimique. Ces mélangeurs comportent une enveloppe externe munie en sortie d'une filière d'extrusion, enveloppe à l'intérieur de laquelle un ou deux arbres sont entraînés en rotation de façon que la structure périphérique d'un arbre coopère avec l'enveloppe externe et, le cas échéant, avec la structure périphérique de l'autre arbre, pour assurer un mélangeage de la matière et son déplacement dans l'enveloppe externe vers la filière d'extrusion. De préférence, l'arbre (ou chacun des arbres) est constitué d'au moins deux manchons successifs dont la partie intérieure s'adapte sur un axe entraîné en rotation et dont la partie extérieure peut avoir diverses structures périphériques ; parmi les structures classiques, on peut citer, d'une part, un filetage de vis hélicoïdale dont le pas conduit la matière traitée de l'entrée vers la sortie du mélangeur (désigné plus loin par "DF"), d'autre part, un filetage de vis hélicoïdale à pas inverse du précédent (désigné plus loin par "CF") qui repousse la matière traitée dans le sens allant de la sortie vers l'entrée du mélangeur, un tel filetage comportant des rainures longitudinales pour assurer le passage de la matière vers la sortie du mélangeur, et enfin, un tronçon multilobé comportant sur toute sa longueur des palettes (ou lobes) disposées côte à côte et angulairement décalées les unes par rapport aux autres. Un tronçon bilobé comporte donc une succession de lobes décalés de 90° les uns par rapport aux autres et est désigné ci-après par "BL". On peut disposer d'un assez grand nombre de manchons à filetage externe pour faire varier le pas, la profondeur et le nombre de filets dans les différentes zones longitudinales successives du mélangeur. De plus, certaines zones longitudinales du mélangeur peuvent être chauffées par un ou plusieurs fourreaux disposés à l'extérieur de l'enveloppe externe. Le chauffage peut être effectué dans chaque fourreau à l'aide d'au moins une résistance électrique ou d'au moins un échangeur de chaleur.

Selon l'invention, l'arbre ou chaque arbre du mélangeur extrudeur-cuiseur comporte, de préférence, au moins un manchon "DF" formant une vis de transport situé du côté de l'alimentation (ou entrée) du mélangeur, au moins un manchon "CF" (dit "à contre-filet") et/ou un manchon "BL" multilobe constituant une zone de malaxage sous pression, et au moins un manchon "DF" formant une vis de transport situé à l'extrémité de sortie du mélangeur. L'appareil peut également comporter au moins un manchon ayant une action de broyage et/ou d'homogénéisation tels qu'un manchon bilobé "BL".

En plus, du fait qu'il permet de produire des compositions de maquillage homogène contenant une forte proportion de poudre légère, l'utilisation d'un mélangeur extrudeur-cuiseur présente de nombreux autres avantages. La fabrication peut se faire en continu et à plus faible température. Par exemple, un "blush" se fabrique classiquement à une température comprise entre 70 et 90° C ; avec un mélangeur extrudeur-cuiseur, on peut le fabriquer à 50° C ou moins. Le fait de travailler à température plus faible permet d'éviter de dégrader les matières premières, par exemple les poudres expansées, et permet d'introduire dans la composition de maquillage des actifs cosmétiques thermosensibles.

De plus, le procédé selon la présente invention est flexible, car on peut facilement faire varier les produits introduits en alimentation ainsi que les débits d'alimentation et, par conséquent, les formulations. On peut facilement également, en fonction de la composition de maquillage désirée, faire varier les paramètres physiques du traitement tels que la pression, notamment par action sur la section de sortie et la vitesse de rotation des arbres, le cisaillement en cours de traitement, notamment par le choix des manchons multilobes et des formes de filets, le malaxage, notamment par le choix des manchons de type "CF", et la température par régulation des fourreaux thermiques au droit des différentes zones du mélangeur.

Les exemples donnés ci-après, à titre illustratif, permettront de mieux comprendre l'invention.

### EXEMPLE 1 - Préparation d'un "blush"

### A) Mélangeur extrudeur utilisé

On opère dans un mélangeur extrudeur-cuiseur à deux vis (type "BC 21" de la société "CLEXTRAL"), dont la structure est celle schématisée ci-dessous :

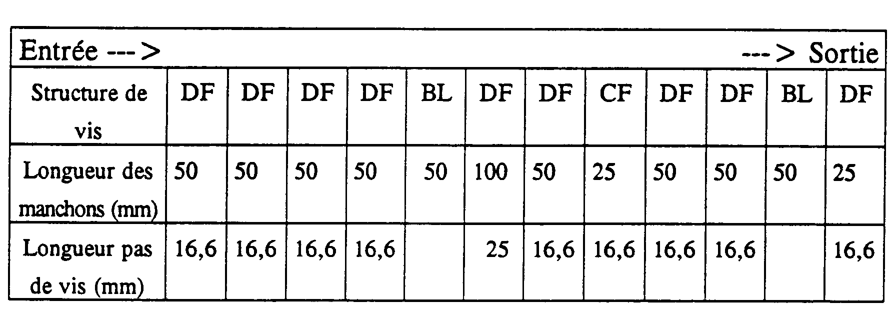

Sur le dessin annexé :
- les figures 1, 3 et 5 représentent en élévation des tronçons de différents types de manchons utilisés sur les arbres du mélangeur mis en oeuvre dans l'exemple 1 ;
- les figures 2, 4 et 6 représentent respectivement des coupes transversales selon II-II, IV-IV et VI-VI des figures 1, 3 et 5.

En se référant au dessin, on voit que l'on a désigné par 1 l'enveloppe externe du mélangeur et par 2a, 2b les axes des deux arbres parallèles, qui y sont disposés. Sur les axes 2a, 2b sont enfilés des manchons adjacents, les deux arbres étant équipés des mêmes manchons sur un même tronçon de la longueur pour coopérer mécaniquement entre eux au cours de la rotation.

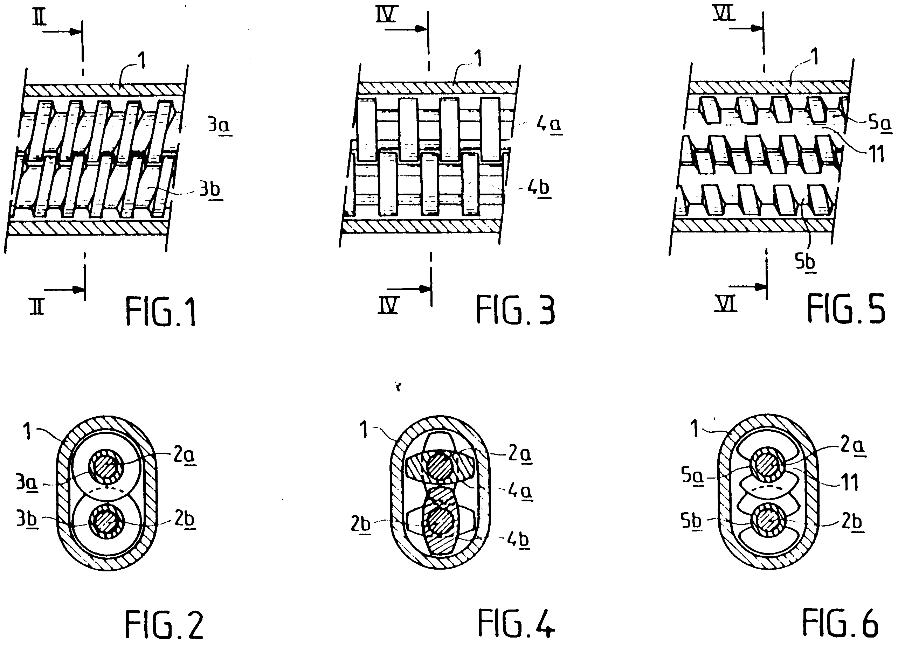

Sur les figures 1 et 2, on a représenté un tronçon où se trouvent des manchons de type "DF" référencés 3a, 3b. Sur les figures 3 et 4, on a représenté un tronçon où se trouvent des manchons 4a, 4b de type bilobé "BL". Sur les figures 5 et 6, on a représenté un tronçon où se trouvent des manchons 5a, 5b de type "CF" avec des rainures longitudinales 11.
Dans le tableau ci-dessus donné :
- DF représente un élément de vis à double filet hélicoïdal, tel qu'illustré sur les figures 1 et 2 ;
- BL représente un élément bilobé, tel qu'illustré sur les figures 3 et 4 ; et
- CF représente un élément de vis à pas inverse de DF, tel qu'illustré sur les figures 5 et 6, comportant des rainures longitudinales 11.
Les différents éléments ont un diamètre externe de 25 mm, un diamètre intérieur de 14 mm ; la distance entre les axes des deux arbres est de 21 mm.
Les deux arbres tournent à la vitesse de 300 t/min ; les orifices de sortie ont une section totale de 500 mm² ; le débit est de 5 kg/h environ.
Le mélangeur est chauffé à 50° C sur toute sa longeur.

### B) Formulation

La formulation est la suivante (poids donnés en g) :

| *Phase grasse :* | |
|---|---|
| Cire de polyéthylène | 4 |
| Cire microcristalline | 1 |
| Vaseline | 7 |
| Néopentanoate d'isostéaryle | 8,5 |
| Huile de paraffine | 15 |
| Octyl dodécanol | 10 |

| *Phase pulvérulente :* | |
|---|---|
| Oxyde de fer jaune | 0,5 |
| Oxyde de fer brun/jaune | 2,2 |
| Oxyde de titane | 5 |
| Laque de calcium du rouge lithol B sur colophane | 0,3 |
| Poudre de nylon | 21 |
| Microsphère ayant une enveloppe en copolymère de vinylidène/acrylonitrile/méthacrylate et renfermant de l'isobutane ("Expancel®" densité 0,036) | 8,5 |
| Para hydroxybenzoate de propyle | 0,2 |
| *Parfum* | 0,3 |

La phase pulvérulente est alimentée en tête de la vis ; la phase grasse est pompée puis alimentée à 100 mm de la tête ; les orifices de sortie ont une section totale de 314 mm² ; le parfum est introduit grâce à une pompe péristaltique vers la fin de la vis.

On obtient un "blush" rouge orangé d'aspect très homogène, très doux au toucher et ayant une texture originale agréable.

A titre de comparaison, on a effectué le mélange de la même composition dans un pétrin "VMI" commercialisé par la société "KENWOOD", dans un mélangeur de type "BAKER" et dans un agitateur à couteaux de type "STEPHAN". A l'oeil nu, on voit que le mélange obtenu n'est pas homogène. Avec une cuve munie d'une turbine à pales contre-rotatives "BROGLIE", il n'a pas été possible de réaliser un tel mélange.

### EXEMPLE 2 - Préparation d'un fond de teint

On opère dans le même mélangeur extrudeur-cuiseur que pour l'exemple 1 avec la structure de vis suivante :

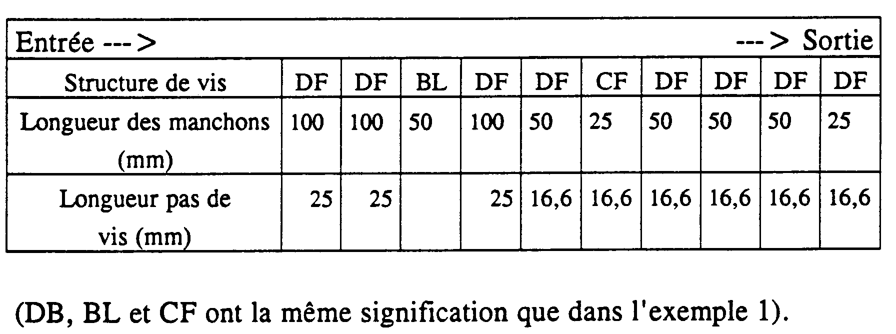

Les éléments ont les mêmes dimensions radiales que dans l'exemple 1 et les axes ont le même entre-axe et la même vitesse de rotation ; les orifices de sortie ont une section totale de 48 mm² ; le débit d'alimentation est également de 5 kg/h environ.

La formulation de la composition est la suivante (poids donnés en g) :

| *Phase grasse* | |
|---|---|
| Palmitate d'éthyl-2 hexyle | 12,2 |
| Isoparaffine hydrogénée | 19,7 |
| Lanolate d'isopropyle stabilisé | 5,8 |
| Parahydroxybenzoate de propyle | 0,3 |
| Parfum | 0,3 |
| Cire micro-cristalline | 10 |
| Cire de carnauba | 6 |

| *Phase pulvérulente* | |
|---|---|
| Oxyde de fer jaune | 2,1 |
| Oxyde de fer brun/jaune | 0,8 |
| Oxyde de fer noir | 0,3 |
| Oxyde de titane | 12 |
| Poudre de nylon 12 | 17,2 |
| Silicate de magnésium | 7 |
| Microsphère ayant une enveloppe en copolymère (vinylidène/acrylonitrile/méthacrylate) et renfermant de l'isobutane ("Expancel®" densité 0,036) | 6 |

La phase grasse et la phase pulvérulente sont introduites en tête de la vis grâce à une pompe péristaltique et à un doseur pondéral.

Le fond de teint obtenu est brun doré, très doux au toucher et très homogène à l'oeil nu.

## Revendications

1. Composition solide ou pâteuse de maquillage, obtenue sous forme homogène par extrusion, contenant une phase grasse et une phase pulvérulente, la phase pulvérulente étant au moins partiellement constituée d'une poudre légère, caractérisée par le fait que la poudre légère a une densité au plus égale à 0,07 et représente de 5 à 30 % en poids par rapport au poids total de la composition, le rapport pondéral de la phase pulvérulente totale à la poudre légère étant compris entre 5 et 16 et que la phase grasse représente de 20 à 70 % en poids par rapport au poids total de la composition.

2. Composition selon la revendication 1, caractérisée par le fait que la poudre légère représente en poids de 5 à 15 % par rapport au poids total de la composition.

3. Composition selon lune des revendications 1 ou 2, caractérisée par le fait que la poudre légère de densité au plus égale à 0,07 comprend au moins un constituant pris dans le groupe formé par des microsphères creuses en matériau thermoplastique et de la poudre de silice.

4. Composition selon la revendication 3, caractérisée par le fait que les microsphères creuses sont en polymères ou copolymères de dérivés éthyléniques, en polyamides, en polyesters, en polymères urée-formaldéhyde ou en copolymères de chlorure de vinylidène.

5. Composition selon l'une des revendications 1 à 4, caractérisée par le fait que la phase pulvérulente contient au moins une poudre ayant une densité plus élevée que celle de la poudre légère, constituée par au moins un pigment et/ou au moins une charge.

6. Composition selon la revendication 5, caractérisée par le fait que le(s) pigment(s) est (sont) choisi(s) dans le groupe formé par des pigments minéraux, des pigments organiques, des pigments nacrés blancs et/ou colorés.

7. Composition selon l'une des revendications 5 ou 6, caractérisée par le fait que les pigments représentent jusqu'à 30 % en poids par rapport au poids total de la composition.

8. Composition selon la revendication 5, caractérisée par le fait que la (les) charge(s) est (sont) choisie(s) dans le groupe formé par le talc, les micas, l'amidon, le kaolin, les oxydes de zinc et de titane, le carbonate de calcium précipité, les savons métalliques dérivés d'acides organiques carboxyliques ayant 8 à 22 atomes de carbone, les poudres de polymères synthétiques, la silice, les poudres organiques, les poudres de silicone, les poudres fluorées.

9. Composition selon l'une des revendications 5 ou 8, caractérisée par le fait que la (les) charge(s) représente(nt) jusqu'à 80 % en poids par rapport au poids total de la composition.

10. Composition selon l'une des revendications 1 à 9, caractérisée par le fait que la phase grasse est constituée par au moins un liant gras, liquide ou solide à température ambiante, et/ou par au moins un polymère synthétique oléosoluble.

11. Composition selon la revendication 10, caractérisée par le fait que le liant est choisi dans le groupe formé par les huiles ou corps gras d'origine végétale, animale, minérale ou synthétique et les cires d'origine végétale, minérale, animale ou synthétique.

12. Composition selon l'une des revendications 1 à 11, caractérisée par le fait qu'elle contient au moins un actif cosmétique lipophile.

13. Composition selon l'une des revendications 1 à 12, caractérisée par le fait que la composition contient au moins un ingrédient choisi dans le groupe formé par les agents astringents, les agents adoucissants, les agents hydratants, les agents cicatrisants, les agents antiradicaux libres, les vitamines et les parfums.

14. Composition selon la revendication 13, caractérisée par le fait que le(s) ingrédient(s) représente(nt) jusqu'à 5 % en poids par rapport au poids total de la composition.

15. Procédé de préparation d'une composition cosmétique homogène selon l'une des revendications 1 à 14 constituée d'une phase grasse et d'une phase pulvérulente contenant de 5 à 30 % en poids de poudre légère, caractérisé par le fait qu'on effectue le mélange dans un mélangeur extrudeur-cuiseur comportant, dans une enveloppe externe (1) munie en sortie d'une filière d'extrusion, un (ou deux) arbre(s) entraîné(s) en rotation de façon que la structure périphérique d'un arbre coopère avec l'enveloppe externe (1) et, le cas échéant, avec la structure périphérique de l'autre arbre pour assurer un mélangeage de la matière et son déplacement dans l'enveloppe externe (1) vers la filière d'extrusion.

16. Procédé selon la revendication 15, caractérisé par le fait que l'(les) arbre(s) est (sont) constitué(s) d'au moins deux manchons successifs (3a, 3b; 4a, 4b ;5a, 5b), dont la partie intérieure s'adapte sur un axe (2a, 2b) entraîné en rotation et la partie extérieure a une structure périphérique différente selon les manchons.

17. Procédé selon l'une des revendications 15 ou 16, caractérisé par le fait que l'arbre (ou chaque arbre) comporte au moins un manchon (3a ou 3b) formant une vis de transport situé du côté de l'alimentation du mélangeur, au moins un manchon (5a ou 5b) à contre-filet et/ou un manchon (4a, 4b) multilobe, et au moins un manchon (3a ou 3b) formant une vis de transport situé à l'extrémité de sortie du mélangeur.

## Claims

1. Solid or pasty make-up composition obtained in homogeneous form by extrusion, containing a fatty phase and a pulverulent phase, the pulverulent phase consisting at least partially of a light powder, characterized in that the light powder has a relative density not exceeding 0.07 and represents from 5 to 30 % by weight relative to the total weight of the composition, the weight ratio of the total pulverulent phase to the light powder being between 5 and 16 and in that the fatty phase represents from 20 to 70 % by weight relative to the total weight of the composition.

2. Composition according to Claim 1, characterized in that the light powder represents from 5 to 15 % by weight relative to the total weight of the composition.

3. Composition according to either of Claims 1 and 2, characterized in that the light powder of relative density not exceeding 0.07 includes at least one constituent taken from the group made up of hollow microspheres made of thermoplastic material and of silica powder.

4. Composition according to Claim 3, characterized in that the hollow microspheres are made of polymers or copolymers of ethylene derivatives, of polyamides, of polyesters, of urea-formaldehyde polymers or of vinylidene chloride copolymers.

5. Composition according to one of Claims 1 to 4, characterized in that the pulverulent phase contains at least one powder which has a density higher than that of the light powder, consisting of at least one pigment and/or at least one filler.

6. Composition according to Claim 5, characterized in that the pigment(s) is(are) chosen from the group made up of white and/or coloured inorganic pigments, organic pigments and pearly pigments.

7. Composition according to either of Claims 5 and 6, characterized in that the pigments represent up to 30 % by weight relative to the total weight of the composition.

8. Composition according to Claim 5, characterized in that the filler(s) is(are) chosen from the group made up of talc, micas, starch, kaolin, zinc and titanium oxides, precipitated calcium carbonate, metallic soaps derived from carboxylic organic acids containing 8 to 22 carbon atoms, powdered synthetic polymers, silica, organic powders, silicone powders and fluorinated powders.

9. Composition according to either of Claims 5 and 8, characterized in that the filler(s) represents (represent) up to 80 % by weight relative to the total weight of the composition.

10. Composition according to one of Claims 1 to 9, characterized in that the fatty phase consists of at least one fatty binder which is liquid or solid at ambient temperature and/or of at least one oil-soluble synthetic polymer.

11. Composition according to Claim 10, characterized in that the binder is chosen from the group made up of oils or fatty substances of vegetable, animal, mineral or synthetic origin and waxes of vegetable, mineral, animal or synthetic origin.

12. Composition according to one of Claims 1 to 11, characterized in that it contains at least one lipophilic cosmetic agent.

13. Composition according to one of Claims 1 to 12, characterized in that the composition contains at least one ingredient chosen from the group made up of astringent agents, softening agents, moisturizing agents, cicatrizing agents, agents against free radicals, vitamins and perfumes.

14. Composition according to Claim 13, characterized in that the ingredient(s) represents(represent) up to 5 % by weight relative to the total weight of the composition.

15. Process for the preparation of a homogeneous cosmetic composition according to one of Claims 1 to 14, consisting of a fatty phase and of a pulverulent phase, containing from 5 to 30 % by weight of light powder, characterized in that the mixing is performed in a cooker-extruder mixer comprising, in an outer enclosure (1) fitted with an extrusion die at the exit, one (or two) shaft(s) driven in rotation so that the peripheral structure of a shaft interacts with the outer enclosure (1) and, if appropriate, with the peripheral structure of the other shaft to ensure mixing of the material and its movement in the outer enclosure (1) towards the extrusion die.

16. Process according to Claim 15, characterized in that the shaft(s) consists(consist) of at least two successive sleeves (3a, 3b; 4a, 4b; 5a, 5b) whose inner part is fitted onto an axle (2a, 2b) driven in rotation and whose outer part has a peripheral structure which differs according to the sleeves.

17. Process according to either of Claims 15 and 16, characterized in that the shaft (or each shaft) comprises at least one sleeve (3a or 3b) forming a conveyor screw situated on the feed side of the mixer, at least one sleeve (5a or 5b) with a counterflight and/or a multilobed sleeve (4a, 4b), and at least one sleeve (3a or 3b) forming a conveyor screw situated at the exit end of the mixer.

## Patentansprüche

1. Homogen extrudierte feste oder pastenartige Schminkzusammensetzung, die eine Fettphase und eine Pulverphase enthält, wobei die Pulverphase mindestens teilweise aus einem leichten Pulver besteht, dadurch gekennzeichnet, daß das leichte Pulver eine Dichte von kleiner oder gleich 0,07 und 5 bis 30 Gew.-% vom Gesamtgewicht der Zusammensetzung aufweist, wobei das Gewichtsverhältnis der gesamten Pulverphase zum leichtem Pulver 5 bis 16 beträgt, und daß die Fettphase 20 bis 70 Gew.-% vom Gesamtgewicht der Zusammensetzung darstellt.

2. Zusammensetzung nach Anspruch 1, dadurch gekennzeichnet, daß das leichte Pulver 5 bis 15 Gew.-% vom Gesamtgewicht der Zusammensetzung darstellt.

3. Zusammensetzung nach einem der Ansprüche 1 oder 2, dadurch gekennzeichnet, daß das leichte Pulver von einer Dichte von höchstens gleich 0,07 mindestens einen Bestandteil aus der Gruppe enthält, die aus hohlen Mikrokugeln aus thermoplastischem Material und Siliciumoxidpulver besteht.

4. Zusammensetzung nach Anspruch 3, dadurch gekennzeichnet, daß die hohlen Mikrokugeln aus Polymeren oder Copolymeren von Ethylenderivaten, aus Polyamiden, aus Polyestern, aus Harnstoff-Formaldehyd-Polymeren oder aus Vinylidenchlorid-Copolymeren bestehen.

5. Zusammensetzung nach einem der Ansprüche 1 bis 4, dadurch gekennzeichnet, daß die Pulverphase mindestens ein Pulver enthält, das eine höhere Dichte als das leichte Pulver hat und aus mindestens einem Pigment und/oder mindestens einem Füllstoff besteht.

6. Zusammensetzung nach Anspruch 5, dadurch gekennzeichnet, daß das/die Pigment/e aus der Gruppe ausgewählt ist/sind, die aus den mineralischen Pigmenten, den organischen Pigmenten und den weißen und/oder farbigen Perlmuttpigmenten besteht.

7. Zusammensetzung nach einem der Ansprüche 5 oder 6, dadurch gekennzeichnet, daß die Pigmente bis zu 30 Gew.-% vom Gesamtgewicht der Zusammensetzung darstellen.

8. Zusammensetzung nach Anspruch 5, dadurch gekennzeichnet, daß der/die Füllstoff/e aus der Gruppe ausgewählt ist/sind, die aus Talk, Glimmern, Stärke, Kaolin, Zink- und Titanoxiden, ausgefälltem Calciumcarbonat, von organischen Carboxylsäuren mit 8 bis 22 Kohlenstoffatomen abstammenden Metallseifen, synthetischen Polymerpulvern, Siliciumoxid, organischen Pulvern, Siliconpulvern und Fluorpulvern besteht.

9. Zusammensetzung nach einem der Ansprüche 5 oder 8, dadurch gekennzeichnet, daß der/die Füllstoffe bis zu 80 Gew.-% vom Gesamtgewicht der Zusammensetzung darstellen.

10. Zusammensetzung nach einem der Ansprüche 1 bis 9, dadurch gekennzeichnet, daß die Fettphase aus mindestens einem bei Raumtemperatur flüssigen oder festen fetten Bindemittel und/oder aus mindestens einem öllöslichen synthetischen Polymer besteht.

11. Zusammensetzung nach Anspruch 10, dadurch gekennzeichnet, daß das Bindemittel aus der Gruppe ausgewählt ist, die aus den pflanzlichen, tierischen, mineralischen oder synthetischen Ölen oder Fetten und den pflanzlichen, mineralischen, tierischen oder synthetischen Wachsen besteht.

12. Zusammensetzung nach einem der Ansprüche 1 bis 11, dadurch gekennzeichnet, daß sie mindestens einen lipophilen kosmetischen Wirkstoff enthält.

13. Zusammensetzung nach einem der Ansprüche 1 bis 12, dadurch gekennzeichnet, daß sie mindestens einen Zusatz enthält, der aus der Gruppe ausgewählt ist, die aus astringierenden Mitteln, weichmachenden Mitteln, anfeuchtenden Mitteln, Wundheilungsmitteln, Mitteln gegen freie Radikale, Vitaminen und Duftstoffen besteht.

14. Zusammensetzung nach Anspruch 13, dadurch gekennzeichnet, daß der Zusatz/die Zusätze bis zu 5 Gew.-% vom Gesamtgewicht der Zusammensetzung darstellt/darstellen.

15. Verfahren zur Herstellung einer homogenen kosmetischen Zusammensetzung nach einem der Ansprüche 1 bis 14, die aus einer Fettphase und einer Pulverphase besteht, die 5 bis 30 Gew.-% leichtes Pulver enthält, dadurch gekennzeichnet, daß man in einem Heiz- und Extrusionsmischer mischt, der in einer an seinem Ausgang mit einer Extrusionsdüse versehenen Außenhülle (1) eine (oder zwei) Wellen aufweist, die so in Drehung versetzt wird(werden), daß die Umfangsstruktur einer Welle mit der Außenhülle (1) und ggf. mit der Umfangs-struktur der anderen Welle zusammenwirkt, um das Material zu mischen und es in der Außenhülle (1) auf die Extrusions-düse zu zu bewegen.

16. Verfahren nach Anspruch 15, dadurch gekennzeichnet, daß die Welle(n) aus mindestens zwei aufeinanderfolgenden Muffen (3a, 3b; 4a, 4b; 5a, 5b) besteht(bestehen), deren innerer Teil auf eine in Drehung versetzte Achse (2a, 2b) aufgepaßt ist und deren äußerer Teil eine je nach Muffe verschiedene Umfangsstruktur hat.

17. Verfahren nach einem der Ansprüche 15 oder 16, dadurch gekennzeichnet, daß die Welle (oder jede Welle) mindestens eine eine Förderschnecke bildende Muffe (3a oder 3b), die auf der Seite der Beschickung des Mischer gelegen ist, mindestens eine Muffe (5a oder 5b) mit Gegengewinde und/oder eine mehrblättrige Muffe (4a, 4b) und mindestens eine eine Förderschnecke bildende Muffe (3a, 3b) aufweist, die am Austrittsende des Mischers angeordnet ist.
